# EUROPÄISCHE PATENTSCHRIFT

(11) **EP 2 618 138 B1**
(45) Veröffentlichungstag und Bekanntmachung des Hinweises auf die Patenterteilung: **04.11.2015**
(21) Anmeldenummer: 13151477.0
(22) Anmeldetag: 16.01.2013
(51) Int. Cl.: G01N 27/22, D06F 39/00, A47L 15/42, G01N 27/30, G01N 33/18

(54) **Sensorvorrichtung zur Erfassung von Flüssigkeitseigenschaften**
Sensor device for detecting fluid properties
Dispositif de capteur destiné à déterminer les propriétés d'un liquide

(30) Priorität: 20.01.2012 DE 202012000569 U
(43) Veröffentlichungstag der Anmeldung: 24.07.2013
(60) Teilanmeldung: 14160557.6
(73) Patentinhaber: Seuffer GmbH & Co. KG, 75365 Calw (DE)
(72) Erfinder: Gruden, Roman, 75180 Pforzheim (DE)
(74) Vertreter: Winter, Brandl, Fürniss, Hübner, Röss, Kaiser, Polte - Partnerschaft mbB

(56) Entgegenhaltungen:
- WO-A1-03/104798
- WO-A2-2011/064770
- DE-A1- 3 812 687
- DE-A1- 10 042 846
- DE-A1- 10 212 494
- DE-A1- 19 755 418
- DE-C1- 19 511 556
- DIETMAR ENDE ET AL: "Impedanzspektroskopie", CHEMIE IN UNSERER ZEIT, Bd. 27, Nr. 3, Juli 1993 (1993-07), Seiten 134-140, XP055095442, DOI: 10.1002/ciuz.19930270305

## Beschreibung

Die vorliegenden Erfindung umfasst eine Sensorvorrichtung zur Erfassung von Flüssigkeitseigenschaften, und insbesondere eine Vorrichtung zur Erfassung von Eigenschaften wässriger Lösungen.

Aus einem Stand der Technik gemäß der Druckschrift DE 196 11 174 C1 ist ein Messwertaufnehmer zur elektrodenlosen Leitfähigkeitsmessung von wässrigen Lösungen, bekannt, bei dem drei Transformatoren verwendet werden. Ein erster Transformator steht in Verbindung mit einer Messflüssigkeit und transformiert eine Generatorwechselspannung in ein Magnetfeld, wobei die Messflüssigkeit von dem durch den ersten Transformator mit der Generatorwechselspannung erzeugten Magnetfeld durchdrungen wird. Es sind ferner ein zweiter und ein dritter Transformator vorgesehen, die beide über das in der Flüssigkeit vorhandene Magnetfeld mit dem ersten Transformator verkoppelt sind. Jeder der beiden weiteren Transformatoren mit der Funktion als Ausgangstransformator wird auf eine unterschiedliche Resonanzfrequenz abgeglichen, sodass die beiden Resonanzfrequenzen gegeneinander versetzt sind. Die Addition der beiden Ausgangsspannungen ergibt einen breitbandigen Frequenzgang unter dem Materialeinfluss der Messflüssigkeit. Die Ausgangssignale der beiden weiteren Transformatoren werden ausgewertet zur Bestimmung der Leitfähigkeit der Messflüssigkeit in Form einer wässrigen Lösung, wobei mittels einer entsprechenden Schaltungsanordnung eingekoppelte Störsignale beseitigt werden.

Die Druckschrift DD 217 557 A1 offenbart ein Verfahren zur Regelung der Reinigungs- oder Spülmittelzugabe in Waschgeräten, wobei in einem Waschgerät zur physikalisch-chemischen Bestimmung der Eigenschaften der Waschlauge unterschiedliche Sensoren angeordnet sind. Über eine elektronische Schaltung, mittels der die Ausgangssignale der Sensoren ausgewertet werden, wird eine Änderung des Anstiegs als Messsignal während der Zugabe von Reinigungs- oder Spülmitteln erfasst und ausgewertet. Es kann hieraus die Waschmitteldosierung für das Waschgerät gesteuert werden.

Ferner offenbart die Druckschrift DE 197 55 418 A1 ein Sensorelement sowie eine Vorrichtung zur Messung komplexer Impedanzen in Materialien, wobei das Sensorelement zwei aus einem leitfähigen Material bestehende Elektroden aufweist, die in einem vorbestimmten Abstand zueinander angeordnet sind. Die beiden Elektroden sind mit einer dünnen Isolierschicht überzogen, die eine im Vergleich zu dem vorbestimmten Abstand geringe Schichtdicke aufweist. Das gebildete Sensorelement ist gegenüber den Umgebungsmedien, deren Eigenschaften erfasst werden sollen, weitgehend unempfindlich. In einer Auswerteschaltung werden die Ausgangssignale der Sensoren einer weiteren Verarbeitung unterzogen, und es kann eine Analyse der Eigenschaften einer jeweils zwischen den Elektroden angeordneten Flüssigkeit durchgeführt werden. Insbesondere können komplexe Impedanzen als Maß für die Eigenschaften der Flüssigkeit bestimmt und ausgewertet werden.

Die Druckschrift DE 195 11 556 C1 offenbart eine Sensoranordnung, wobei im Wesentlichen zwei getrennte Messräume zur Erfassung von Eigenschaften und eines Füllstands derselben Flüssigkeit vorgesehen sind. In jedem der beiden Messräume ist eine kapazitive Sensoreinrichtung in Form eines Zylinderkondensators angeordnet. Im Einzelnen erfolgt eine Messung der Eigenschaften der Flüssigkeit und des Füllstands mit einer Impedanzmessung, wobei in Verbindung mit dem ersten Messraum und der ersten Sensoreinrichtung ein Zustandssignal ermittelt wird, und mit der zweiten Sensoreinrichtung im zweiten Messraum der Füllstand der Flüssigkeit in einem größeren Behälter, in dem die Sensoranordnung eingesetzt ist, erfasst wird. Insbesondere wird die in Verbindung mit dem ersten Messraum ermittelte Zustandsinformation zur Kalibrierung der Impedanzmessung der zweiten Sensoreinrichtung im zweiten Messraum herangezogen.

Die Druckschrift WO 2011/064770 A2 offenbart einen Analysator und ein zugehöriges Verfahren zur elektrischen Spektroskopie einer Flüssigkeit am Beispiel von Milch. Die zu erfassende Flüssigkeit wird hinsichtlich ihrer Eigenschaften überprüft, wobei dies bspw. die Konzentration von Komponenten der Milch sein kann, wie bspw. ein Fettgehalt oder ein Proteingehalt. Hierzu wird die Milch in einem Behälter eingebracht, der in Verbindung steht zu einer kapazitiven Messeinrichtung, bspw. einer Sensoreinrichtung, und wobei in einer auf diese Weise gebildeten Abtastzelle beispielsweise bogenförmige Elektroden vorgesehen sein können. Anstelle gegenüberliegender Elektroden, zwischen denen sich eine Milchprobe befinden kann, können auch in der Nähe des Probenbehälters nebeneinander angeordnete Elektroden vorgesehen sein. In jedem Fall wird mittels der zwischen den Elektroden befindlichen Milchprobe nach Ansteuerung der Elektroden mittels entsprechender Signale mit vorbestimmten Frequenzen eine Eigenschaftsinformation erhalten, die ausgewertet werden kann.

Die Druckschrift DE 38 12 687 A1 offenbart einen kapazitiven Sensor zur Bestimmung eines Füllstands einer Flüssigkeit in einem Behälter, wobei eine Sensoranordnung in Form eines Zylinderkondensators von oben in die Flüssigkeit eintaucht und wobei die sich mit dem Füllstand der Flüssigkeit ändernden Kapazitätswerte der Sensoranordnung bei zwei unterschiedlichen Frequenzen eine Füllstandinformation ergeben. Die Füllstandinformation kann in entsprechender Weise ausgewertet werden. Der Zylinderkondensator mit einer äußeren Ringelektrode und einer inneren Zentralelektrode wird von der Flüssigkeit umströmt, wobei zwischen den Elektroden der jeweils zu erfassende Flüssigkeitspegel angeordnet ist:

Schließlich offenbart die Druckschrift DE 102 12 494 A1 eine Vorrichtung zur kapazitiven Messung eines Mischungsverhältnisses eines Mediums, wobei ein Medium erfasst wird, das aus zwei Komponenten unterschiedlicher Dielektrizitätskonstanten besteht. Von einer Trägerplatte ragen voneinander elektrisch isolierte erste und zweite Elektroden ab, wobei die erste Elektrode ein sich axial erstreckender Hohlkörper ist, in den durch entsprechende Öffnungen das zu erfassende Medium eindringen kann. Die zweite Elektrode ist koaxial im Inneren der ersten Elektrode angeordnet, so dass sich eine Sensoreinrichtung in Form eines Zylinderkondensators ergibt. Das den Zwischenraum zwischen den beiden Elektroden durchströmende Medium bildet ein Dielektrikum des Zylinderkondensators, dessen Änderung beispielsweise im Vergleich zu gespeicherten Referenzwerten nach einer entsprechenden Auswertung erfasst werden kann.

Entsprechend den vorstehend angegebenen bekannten Möglichkeiten zur Erfassung der Eigenschaften von beispielsweise einer Waschlauge oder sonstigen fluiden Medien können mit den dargestellten Vorrichtungen und einer vergieichsinreise komplizierten Auswertung von Messergebnissen Parameter der Eigenschaften der zu untersuchenden Medien bestimmt werden. Insbesondere besteht allgemein ein großer Bedarf an der Erfassung von-Materialeigenschaften wie beispielsweise einer Ölqualität in einer Brennkraftmaschine, um gezielt einen Ölwechsel bei verschlechterten Eigenschaften des verwendeten Öls vorzunehmen. Es kann ebenfalls eine Bremsflüssigkeit in Kraftfahrzeugen oder ein allgemeines Hydrauliköl hinsichtlich seiner im Betrieb veränderlichen Eigenschaften überprüft werden.

Die vorstehend angegebenen bekannten Möglichkeiten zur Erfassung der Eigenschaften von Flüssigkeiten, wie beispielsweise einer Waschlauge oder sonstigen wässrigen Lösungen, stellen Vorrichtungen und Systeme dar, mittels denen in Verbindung mit einer vergleichsweise komplizierten Auswertung der Messergebnisse und aufwändigen Sensoreinrichtungen die Eigenschaften oder zumindest einzelne Parameter der jeweiligen zu untersuchenden Flüssigkeiten bestimmt werden.

Im Hinbtick auf das Bestreben zu einem umweltfreundlichen und gleichzeitig optimierten Waschvorgang besteht allgemein ein großer Bedarf an der Erfassung der Eigenschaften einer Waschlauge in einer Wascheinrichtung zu Beginn und im gesamten Verlauf des Waschvorgangs, sodass in Richtung einer geringeren Umweltverschmutzung beispielsweise die eingesetzte Wassermenge und/oder Waschmittelmenge genau bestimmt und ferner die Waschmittelmenge in Abhängigkeit von dem Verschmutzungsgrad zum Reinigen der Wäsche oder bestimmten Wassereigenschaften (Mineralgehalt, Wasserhärte) genau dosiert werden kann.

Eine gezielte und richtige Waschmitteldosierung führt einerseits zur Einsparung von Energie und Wasser, sowie andererseits zu der erstrebten geringeren Belastung von Abwässern. Eine Erfassungsvorrichtung zur Erfassung einer Waschlauge in einer Wascheinrichtung, beispielsweise hinsichtlich der Waschmittelkonzentration oder eines Verschmutzungsgrads, sollte einfach aufgebaut und betriebssicher ausgeführt sein. Da zukünftig immer mehr Wascheinrichtungen in privaten Haushalten und in der Industrie mit einer Erfassungsvorrichtung zur Erfassung der Eigenschaften der Waschlauge ausgerüstet werden sollen, besteht ein großer Bedarf an einfach aufgebauten Sensorvorrichtungen, die in größeren Stückzahlen kostengünstig hergestellt werden können, und die gleichwohl ein verlässliches Erfassungsergebnis zur weiteren Auswertung bereitstellen können. Im Idealfall sollte jede Wascheinrichtung mit einer derartigen Erfassungseinrichtung oder Sensorvorrichtung ausgerüstet werden, sodass auch die Erfordernisse einer unkomplizierten Montage während des Herstellungsvorgangs der Wascheinrichtung in Verbindung mit dem einfachen Aufbau zu berücksichtigten sind.

Der vorliegenden Erfindung liegt daher die Aufgabe zu Grunde, eine Sensorvorrichtung zur Erfassung von Flüssigkeitseigenschaften derart auszugestalten, dass bei einem einfachen Aufbau und einer damit verbundenen kostengünstigen Herstellung einerseits eine verlässliche Erfassung der Eigenschaften einer Flüssigkeit und andererseits eine Möglichkeit zur Änderung der elektrischen Eigenschaften der Sensorvorrichtung gewährleistet ist.

Erfingdungsgemäß wird diese Aufgabe mit den in den Patentansprüchen angegeben Merkmalen gelöst.

Die erfindungsgemäße Sensorvorrichtung zur Erfassung von Eigenschaften fluider Medien in einem Behälter umfasst zumindest eine aus einem isolierenden Material gebildeten Grundplatte mit einer ersten dem Medium ausgesetzten Oberfläche und zumindest einem als Zylinderkondensator ausgebildeten Sensorelement mit zumindest einer äußeren Elektrode und einer teilweise von der äußeren Elektrode umgebenen inneren Elektrode, wobei sich die zumindest eine äußere und die innere Elektrode von der ersten Oberfläche der Grundplatte erstrecken und wobei die Sensorvorrichtung dem zu erfassenden Medium zumindest teilweise ausgesetzt ist und das Medium zwischen die äußere und innere Elektrode gelangt. Die zumindest eine äußere Elektrode und die innere Elektrode sind in einer Erstreckungsrichtung in eine Mehrzahl gegeneinander isolierter Elektrodenabschnitte aufgeteilt, und es sind die äußere und die innere Elektrode in gleicher Weise aufgeteilt. Die Elektrodenabschnitte der äußeren und inneren Elektroden sind jeweils mit Anschlussleitungen verbunden und individuell und unabhängig voneinander ansteuerbar.

Die vorliegenden Erfindung betrifft somit eine Sensorvorrichtung zur Erfassung von Materialeigenschaften, und im vorliegenden Fall zur Erfassung von Eigenschaften einer Flüssigkeit, wie beispielsweise einer Waschlauge oder anderen wässrigen Lösungen, wobei die Sensorvorrichtung im Wesentlichen als ein kapazitiver Sensor aufgebaut ist. Entsprechende Elektroden sind auf einer Seite einer isolierenden Grundplatte zueinander beabstandet und isoliert angeordnet.

Mit der erfindungsgemäßen Anordnung der Sensorvorrichtung ist es möglich, unterschiedliche Eigenschaften eines interessierenden Mediums, wie beispielsweise einer Waschlauge, und anderer wässriger Lösungen in zuverlässiger Weise zu erfassen, wobei die Sensorvorrichtung gleichzeitig einen einfachen Aufbau aufweist. Mittels des einfachen Aufbaus besteht die Möglichkeit einer kostengünstigen Herstellung. Dies ist insbesondere bei der Fertigung der Sensorvorrichtung in größeren Stückzahlen für eine Serie von Wascheinrichtungen oder weiteren Anwendungen vorteilhaft.

Die Sensorvorrichtung ermöglicht die genaue Erfassung von Eigenschaften von wässrigen Lösungen, und kann in Folge der kostengünstigen Herstellung auch bei größeren Stückzahlen beispielsweise in Wascheinrichtungen für private Haushalte und die Industrie eingesetzt werden. Es ist ebenfalls möglich, mittels der erfindungsgemäßen Sensorvorrichtung eine Veränderung der Eigenschaften des interessierenden fluiden Mediums, wie beispielsweise Frischwasser (Mineralgehalt, Wasserhärte) oder auch von Abwässern (sich ändernder Verschmutzungsgrad) zu erfassen. Verschiedene Ausführungsformen werden verschiedenen Anwendungen oder Einsatzbereichen gerecht. Es besteht mit diesen Sensorvorrichtungen die Möglichkeit, gleichzeitig oder in vorbestimmter Weise sequenziell mehrere Eigenschaften (interessierende Parameter) des fluiden Mediums zu erfassen. Allen Ausführungsformen liegt jedoch ein Aufbau zugrunde, der bei einer verlässlichen Erfassung kostengünstig hergestellt und eingesetzt werden kann.

Weitere Ausgestaltungen sind in den Unteransprüchen angegeben.

Die zumindest eine äußere und die innere Elektrode kann in eine Vielzahl gleicher oder unterschiedlicher Elektrodenabschnitte aufgeteilt sein, und die bei der ungleichen aufteilung gebildeten Elektrodenabschnitte können hinsichtlich ihrer Ausdehnung in der Erstreckungsrichtung zueinander in vorbestimmten Verhältnissen aufgeteilt sein.

Des Weiteren können die einzelnen Elektrodenabschnitte aus einem gleichen oder unterschiedlichen leitenden Material gebildet sein, wobei jeweils einander entsprechende Elektrodenabschnitte der äußeren und der inneren Elektroden aus einem gleichen Material gebildet sein können.

Die Erfindung wird nachstehend anhand von Ausführungsbeispielen unter Bezugnahme auf die Zeichnung näher beschrieben. Es zeigen:
Figur 1 ein Blockschaltbild einer Schaltungsanordnung zur Durchführung von Messungen in Verbindung mit einer nicht erfindungsgemäßen Sensorvorrichtung,
Figur 2 eine perspektivische Darstellung der Sensorvorrichtung nach Figur 1,
Figur 3 eine schematische Darstellung der Sensorvorrichtung nach Figur 1 gemäß einem ersten nicht erfindungsgemäßen Grundlagenbeispiel,
Figur 4 eine schematische Darstellung der Sensorvorrichtung nach Figur 1 gemäß einem zweiten nicht erfindungsgemäßen Grundlagenbeispiel,
Figur 5 eine schematische Darstellung der Sensorvorrichtung nach Figur 1 gemäß einem dritten nicht erfindungsgemäßen Grundlagenbeispiel,
Figur 6 eine schematische Darstellung einer Sensorvorrichtung gemäß einem erfindungsgemäßen Ausführungsbeispiel,
Figur 7 eine schematische Darstellung von Teilen der Sensorelement gemäß Figur 6 in einer ersten Variante, und
Figur 8 eine schematische Darstellung von Teilen der in Figur 6 gezeigten Sensorvorrichtung gemäß einer zweiten Variante.

### Beschreibung von Grundlagenbeispielen und einem bevorzugten Ausführungsbeispiel

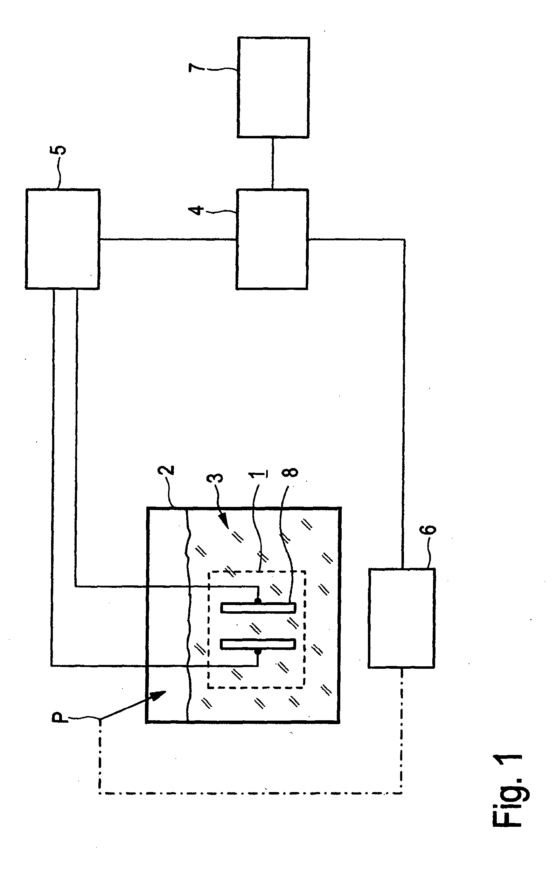

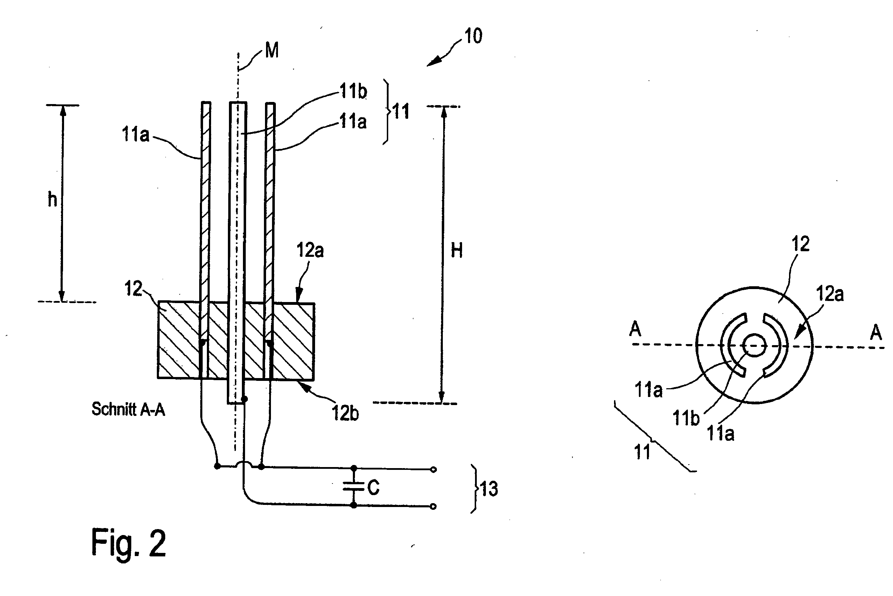

Nachstehend werden anhand des in Figur 1 gezeigten schematischen Blockschaltbilds der grundlegende Aufbau, die Wirkungsweise und die entsprechende Ansteuerung einer Sensorvorrichtung 1 beschrieben. Die Sensorvorrichtung 1 ist gemäß Figur 1 ist lediglich schematisch in vereinfachter Form dargestellt als ein kapazitiver Sensor mit zwei symbolischen Kondensatorplatten, während die weiteren Figuren 2 bis 4 konkrete Ausgestaltungen der Sensorvorrichtung 1 zeigen. Diese werden nachstehend noch beschrieben.

Figur 1 zeigt zur Veranschaulichung des Einsatzes der Sensorvorrichtung 1 zur Erfassung von Eigenschaften eines flüssigen Mediums, wie beispielsweise einer wässrigen Lösung, ein schematisiertes Blockschaltbild mit verschiedenen Komponenten, die zur Durchführung von Messungen geeignet sind und mittels denen die Sensorvorrichtung 1 angesteuert wird.

Im Einzelnen ist gemäß Figur 1 die Sensorvorrichtung 1 in einem Behälter 2 angeordnet, in welchem sich ein fluides Medium 3 befindet. Der Begriff des Behälters 2 ist in allgemeiner Form aufzufassen, wobei es sich um einen Tank in einem Gerät oder einer Maschine, eine Trommel einer Wascheinrichtung (Waschmaschine) oder dergleichen handeln kann. In jedem Fall beinhaltet der Behälter 2 das zu erfassende Medium 3, vorzugsweise eine wässrige Lösung, in einer Ausreichenden Menge, sodass mittels der Sensorvorrichtung 1 die Eigenschaften des Mediums 3 erfasst werden können. Zumindest kann mittels der Sensoreinrichtung 1 und ihrer entsprechenden Ansteuerung ein interessierender Parameter gemessen werden. Die Erfassung kann dabei kontinuierlich oder in vorbestimmten zeitlichen Abständen oder bei Bedarf erfolgen. Eine Steuerung mittels eines EDV-Programms ist möglich.

Die Sensorvorrichtung 1 ist zur Durchführung einer verlässlichen Messung ganz oder zumindest teilweise von dem Medium 3 umgeben. Die Sensorvorrichtung 1 ist in der nachfolgenden Beschreibung als Beispiel in einer Waschlauge (Medium 3) einer Wascheinrichtung (Waschmaschine) angeordnet. Mittels der Sensorvorrichtung 1 wird die Waschlauge hinsichtlich beispielsweise der Konzentration eines Waschmittels oder eines Verschmutzungsgrades überprüft. Darüber hinaus können weitere Parameter der zu erfassenden wässrigen Lösung bestimmt werden. Beispielsweise können die Eigenschaften von Frischwasser (beispielsweise Schadstoffe oder Wasserhärte) bestimmt werden, oder es kann in Abhängigkeit von den erfassten Eigenschaften eines Frischwassers ein Waschvorgang in Verbindung mit einem Waschmittelverbrauch gesteuert oder geregelt werden.

Zur Durchführung einer entsprechenden Messung ist die Sensorvorrichtung 1 beilspielsweise mit einer Steuerungseinrichtung 4 verbunden, die einerseits zur Steuerung der gesamten Abläufe der Erfassung der Eigenschaften des Mediums 3 dient und anderseits eine Ansteuerung der Sensorvorrichtung 1 veranlasst, sowie eine Auswertung der von der Sensorvorrichtung 1 abgegebenen Signale (Erfassungssignale) durchführt. Zu diesem Zweck ist die Steuerungseinrichtung 4 mit einem Ansteuerungsteil 5 verbunden, der in Abhängigkeit von Anweisungen und Befehlen der Steuerungseinrichtung 4 die Sensorvorrichtung 1 mit entsprechenden elektrischen Signalen ansteuert. Beispielsweise werden an die Sensorvorrichtung 1 (d. h. an die Sensorvorrichtungen gemäß den nachstehend beschriebenen Ausführungsbeispielen) zur Durchführung der entsprechenden Messungen elektrische Spannungen in Verbindung mit vorbestimmten Frequenzen oder Frequenzbereichen angelegt. Hierbei kann ein Verfahren in Verbindung mit der Impedanzspektroskopie zur Anwendung kommen.

Die Steuerungseinrichtung 4 ist auch vorgesehen zur Erfassung der Temperatur des Mediums 3 und kann hierzu mit einer in den Figuren nicht gezeigten und im Behälter 2 angeordneten Temperaturerfassungseinrichtung verbunden sein. Die Sensorvorrichtung 1 und der Ansteuerungsteil 5 können hinsichtlich ihrer Funktion auch zusammengefasst und in einer Einrichtung angegeben werden, wobei diese beispielsweise als eine Messeinrichtung bezeichnet werden kann. Die Steuerungseinrichtung 4 ist ferner mit einer Hauptsteuereinheit 6 verbunden, die direkt im Zusammenhang mit beispielsweise der Wascheinrichtung steht, in der der Behälter 2 angeordnet ist. In dem Fall, dass der Behälter 2 der Laugenbehälter einschließlich der Trommel der Wascheinrichtung ist, stellt die Hauptsteuereinheit 6 die eigentliche elektronische Steuerungseinheit der Wascheinrichtung dar.

In der Wascheinrichtung werden in der entsprechenden und programmierten Weise Wassermenge und Waschmittelzulauf sowie der Motorenbetrieb gesteuert. Die Steuerungseinrichtung 4 kann in Abhängigkeit von den Messergebnissen in Verbindung mit dem Betrieb der Sensorvorrichtung 1 die Hauptsteuereinheit 6 dahingehend beeinflussen, dass entsprechend der gewonnen Information und beispielsweise abweichend von einem vorbestimmten Programm eine größere oder kleinere Wassermenge oder eine größere oder kleinere Waschmittelmenge eingesetzt oder auch eine Veränderung der Waschtemperatur vorgenommen werden kann. Es kann mittels der Steuerungseinrichtung 4 in Verbindung mit der Hauptsteuereinheit 6 eine variable Steuerung oder auch eine Regelung im Gesamtsystem der Wascheinrichtung vorgenommen werden. Die allgemeine Einflussnahme der Hauptsteuereinheit 6 auf den Waschvorgang ist in Figur 1 symbolisch mittels einer gestrichelten Linie und einem Pfeil P in Richtung des Behälters 2 dargestellt.

Die Steuerungseinrichtung 4 kann ferner in Verbindung mit einer Speichereinrichtung 7 stehen, in der zur Durchführung der entsprechenden Messungen, d. h. zur Erfassung der Materialeigenschaften des Mediums 3 entsprechende Daten und Programme gespeichert werden können, auf die die Steuerungseinrichtung 4 bedarfsweise zugreifen kann uns wobei auch Erfassungsdaten oder verarbeitete Daten in der Speichereinrichtung 7 gespeichert werden können.

Bei der Durchführung von Messungen mittels der Sensorvorrichtung 1 können gemäß der vorstehenden Beschreibung der Sensorvorrichtung 1 auf eine entsprechende Anweisung der Steuerungseinrichtung 4 mittels des Ansteuerungsteils 5 vorbestimmte physikalische Größen, wie Strom, Spannung in Verbindung mit entsprechenden Frequenzen zugeführt werden. In Verbindung mit der durchgeführten Messung werden durch die Steuerungseinrichtung 4 über den Ansteuerungsteil 5 die Erfassungssignale der Sensorvorrichtung 1 aufgenommen und verarbeitet, gegebenenfalls in Verbindung mit aus der Speichereinrichtung 7 ausgelesenen Programmen oder Daten. Beispielsweise kann ein Vergleich mit Grunddaten hinsichtlich der Eigenschaften des Erfassten Mediums 3 durchgeführt werden, um absolute oder relative Änderungen zu erfassen, wobei auch auftretende Änderungen während vorbestimmter länger andauerhder Vorgänge erfasst werden können.

In Abhängigkeit von dem Erfassungsergebnis, beispielsweise entsprechend einer Konzentration der erfassten Waschlauge in der Wascheinrichtung, kann die Hauptsteuereinheit 6 angewiesen werden, bestimmte Betriebsparameter zu ändern oder beizubehalten, und es können zum späteren Nachvollziehen von Messergebnissen und Abläufen der Wascheinrichtung die Messergebnisse in der Speichereinrichtung 7 gespeichert werden.

Während in der vorstehend beschriebenen Figur 1 die Anordnung der Sensorvorrichtung 1 innerhalb eines Messsystems oder einer Steuerungs- und Regelungssystems angegeben ist, zeigen die weiteren Figuren 2 bis 4 entsprechenden Ausführungsbeispiele der Sensorvorrichtung 1, die an die Stelle der Sensorvorrichtung 1 gemäß Figur 1 eingesetzt und im entsprechender Weise betrieben werden kann.

### Erstes Grundlagenbeispiel

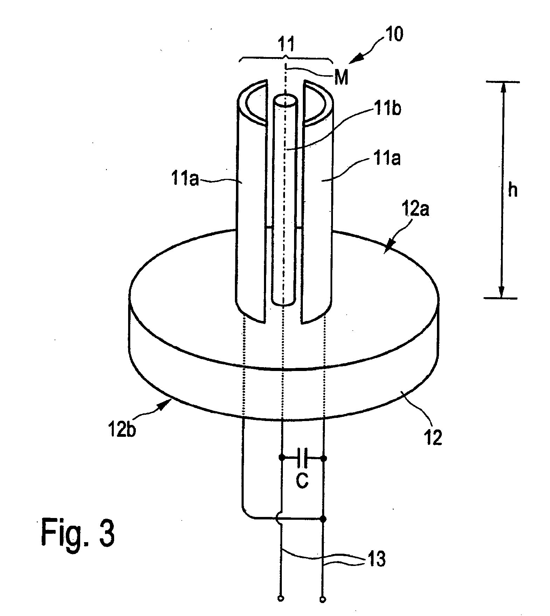

Die Figuren 2 und 3 zeigen ein erstes Grundlagenbeispiel der Sensorvorrichtung 10. Figur 2 zeigt eine Draufsicht auf die Anordnung der Sensorvorrichtung 10, sowie eine Schnittdarstellung entlang einer Schnittlinie A-A. Figur 3 zeigt eine perspektivische Darstellung der Sensorvorrichtung 10.

In der Darstellung gemäß Figur 1 ist die symbolische Sensorvorrichtung 1 als ein kapazitiver Sensor vereinfacht und schematisch durch zwei einander gegenüberliegende Kondensatorplatten 8 dargestellt. Die schematischen Kondensatorplatten veranschaulichen das Grundprinzip eines Plattenkondensators, bei dem zwischen den Platten ein Material mit vorbestimmten und/oder zu erfassenden dielektrischen Eigenschaften angeordnet ist. Im vorliegenden Fall gemäß Figur 1 und in Verbindung mit den Betrachtungen bei einer Wascheinrichtung wird das dielektrische Material durch das Medium 3, und beispielsweise durch eine wässrige Lösung wie eine Waschlauge gebildet.

Die Sensorvorrichtung 10 des ersten Grundlagenbeispiels umfasst gemäß der Darstellung in den Figuren 2 und 3 eine Sensoranordnung, bei der Elektroden (anstelle der schematischen Kondensatorplatten 8 gemäß Figur 1) in Form der einzelnen Elektroden 11 einander gegenüberliegend angeordnet sind. Insbesondere sind die Elektroden 11 der Sensorvorrichtung 10 gemäß dem ersten Grundlagenbeispiel derart angeordnet, dass zumindest eine äußere (erste) Elektroden 11a und im konkreten Fall der Figuren 2 und 3 zwei äußere Elektroden 11 a, die als ein Teil einer Mantelfläche eines Hohlzylinders ausgebildet sind, einer im wesentlichen im Mittelpunkt der Grundfläche des Hohlzylinders angeordneten inneren (zweiten) Elektrode 11b gegenüberstehen. Jede der äußeren Elektroden 11a ist kleiner als die Hälfte der Mantelfläche des Hohlzylinders, so dass zwei voneinander getrennte und beabstandete Elektroden in einer Anordnung ähnlich der von Halbschalen gebildet werden, zwischen denen ein Zwischenraum in vorbestimmter Weise angeordnet ist. Die innere Elektrode 11b verläuft bzw. erstreckt sich entlang der Hauptachse oder Symmetrieachse des gedachten Hohlzylinders, der durch die beiden äußeren Elektroden 11a aufgespannt wird. Sämtliche Elektroden 11 sind in einer aus isolierendem Material bestehenden Grundplatte 12 angeordnet und mechanisch stabil befestigt, sodass ein dielektrisches Material und insbesondere das zu erfassende Medium 3 zwischen die äußeren Elektroden 11a und die innere Elektrode 11b gelangen kann, die sich im Wesentlichen senkrecht von der Grundplatte 12 weg erstrecken. Es werden somit die erste und die zweite Elektrode 11a und 11b von dem Medium 3 umströmt.

Die beiden äußeren Elektroden 11a sind zueinander in vorbestimmter und bevorzugt konstanter Weise beabstandet, sodass zwischen einer der äußeren Elektroden 11a und der inneren Elektroden 11 b ein annähernd konstanter Abstand gebildet wird, und die jeweilige Länge der Elektroden 11 außerhalb der Grundplatte 12 etwa gleich ist. Sämtliche Elektroden 11 verlaufen somit entlang der Mittelachse des gedachten Holzylinders auf einer der Seiten der Grundplatte 12, gemäß den Darstellungen in den Figuren 2 und 3 auf der oberen Seite der Grundplatte 12. Die Seite der Grundplatte 12, auf der die Elektroden 11 angeordnet sind, wird als erste Seite oder als erste Oberfläche 12a bezeichnet.

Auf der anderen Seite der Grundplatte 12, d. h. auf der zweiten Seite oder Oberfläche 12b sind die jeweiligen Elektroden 11 mit elektrischen Anschlussleitungen 13 verbunden. Hierbei stellt die zumindest eine äußere Elektrode 11a den einen Pol dar. Im Falle der beiden äußeren (ersten) Elektroden 11 a sind diese elektrisch miteinander verbunden und stellen denselben Pol der Elektrodenanordnung gemäß den Figuren 2 und 3 dar. Die innere (zweite) Elektrode 11b stellt den anderen Pol dar und ist ebenfalls mit einer der Anschlussleitungen 13 verbunden.

Figur 2 zeigt schematisch die Verbindung der entsprechenden Anschlussleitungen 13 mit den jeweiligen Elektroden 11. Hierbei ist zwischen dem einen Pol der äußeren Elektrode 11 a und dem anderen Pol der inneren Elektrode 11 b ein optionaler Kondensator C angegeben. Der Kondensator C dient zur Festlegung des Frequenzbereichs der Ansteuerungssignale, mittels denen die Sensorvorrichtung 10 angesteuert wird. Mit dem vorbestimmten Kapazitätswert des Kondensators C kann die Frequenz von bestimmten Messpunkten in einem Messablauf festgelegt werden. Die jeweiligen Anschlussleitungen 13 sind zur Durchführung entsprechender Messungen oder Erfassungen mit dem Ansteuerungsteil 5 (Figur 1) verbunden.

Gemäß den Figuren 2 und 3 ist die Grundplatte 12 als eine annähernd kreisförmig Scheibe mit einer vorbestimmten Dicke dargestellt, in deren Mitte die innere Elektrode 11b und um die innere Elektrode 11b mit einem vorbestimmten Abstand die zumindest eine und vorzugsweise beiden äußeren Elektroden 11a angeordnet sind. Die Ausführung der Grundplatte 12 als eine kreisförmige Scheibe stellt lediglich ein Beispiel dar, und ist nicht auf diese Grundform festgelegt. Vielmehr können beliebige Formen der Grundplatte 12 auch mit einer unterschiedlichen Dicke vorgesehen sein, wobei auch die Anordnung der Elektroden 11 nicht notwendigerweise in der Mitte einer symmetrisch ausgebildeten Grundplatte 12 sein muss. In jedem Fall wird die Grundplatte 12 in eine entsprechende Öffnung in dem Behälter 2 eingesetzt, wobei entsprechende Abdichtungsmaßnahmen im Bereich der Öffnung vorgesehen sind zur Vermeidung eines unerwünschten Austretens des Mediums 3 aus dem Behälter 2 an der Einsetzstelle der Sensorvorrichtung 10. Die Sensorvorrichtung 10 befindet sich vorzugsweise im unteren Teil des Behälters 2 und weiter bevorzugt an einer tiefsten Stelle des Behälters 2, sodass auch bei kleinen Mengen des Mediums 3 eine zuverlässig Erfassung der Eigenschaften des Mediums 3 gewährleistet ist.

Die Eigenschaften des zu erfassenden Mediums betreffen beispielsweise eine Konzentration an Mineralien, an waschaktiven Substanzen sowie eine in Folge des Waschvorgangs aufgetretene und auch zunehmende Verschmutzung der Waschlauge. Es kann zumindest ein Parameter wie eine Konzentration bestimmter Substanzen ermittelt werden. Des Weiteren kann an der Sensorvorrichtung 10, insbesondere im Bereich der Grundplatte 12 eine Temperaturerfassungseinrichtung vorgesehen sein.

In den Figuren 2 und 3 ist bei der Darstellung der Sensorvorrichtung 10 eine erste Höhe h und eine zweite Höhe H angegeben. Die erste Höhe h betrifft die freie Länge der Elektroden 11 außerhalb der Grundplatte 12 im zu erfassenden Bereich des Behälters 2. Dieser Bereich der Elektroden ist damit vom Medium 3 umströmt.

Die zweite Höhe H stellt die Gesamtlänge der längsten Elektrode 11, beispielsweise der inneren Elektrode 11b dar, wobei ein Teil der inneren Elektrode 11b oberhalb und ein weiterer Teil unterhalb der Grundplatte 12 angeordnet ist. Der Teil unterhalb der Grundplatte 12 erstreckt sich somit weg von der zweiten Oberfläche 12b. Mit dem unterhalb der Grundplatte 12 liegenden Teil sind die Anschlussleitungen 13 und der Kondensator C verbunden.

Gemäß der Darstellung in den Figuren 2 und 3 besteht die äußere Elektrode 11a aus zwei Teilen, die entsprechend ihrer Form als Teil einer Hohlzylinderwand konzentrisch um die innere Elektrode 11b angeordnet sind. Es besteht ebenfalls die Möglichkeit, die konzentrisch um die innere Elektrode 11 b angeordneten äußeren Elektroden 11a in Form von drei oder mehr etwa gleichartigen Teilen vorzusehen, die jeweils in einer Richtung senkrecht zu einer Grundplatte 12 entlang der Mittellinie M entsprechend zueinander beabstandet sind, sodass eine ungehinderte Strömung des Mediums 3 (Fluide) zwischen den einzelnen Elektrodenteilen auch bei im Verlauf des Waschvorgangs entstandenen Schwebstoffen gewährleistet ist. Sind beispielsweise zwei Paare von äußeren Elektroden 11a vorgesehen, so können zwei der vier größeren Elektroden 11a isoliert (d. h. mit einer Isolierschicht versehen) und die beiden anderen in einer nicht-isolierten Weise gegenüber dem Medium 3 im Behälter 2 ausgeführt sein. Sämtliche Teile der ersten bzw. äußeren Elektrode 11a stellen denselben Pol dar.

Die zumindest eine erste (äußere) Elektrode 11a kann alternativ auch einstückig als vollständiger Hohlzylinder ausgebildet werden, in dessen Innenraum die zweite (innere) Elektrode 11 b im Wesentlichen entlang einer Symmetrieachse oder Mittellinie (M gemäß Figur 3) verläuft. Die erste Elektrode 11a in der Form eines Hohlzylinders weist einige Öffnungen in ihrer Zylinderwand auf, so dass das zu erfassende Medium 3 die beiden Elektroden 11 (11a und 11b) umströmen kann und insbesondere in den Raum zwischen den Elektroden 11 gelangen kann. Abweichend von der Darstellung der Draufsicht in Figur 2 ergibt sich dann ein vollständiger Hohlzylinder mit entsprechenden Strömungsöffnungen, mit denen eine stetige Umströmung des Bereichs zwischen den Elektroden 11 gewährleistet ist.

### Zweites Grundlagenbeispiel

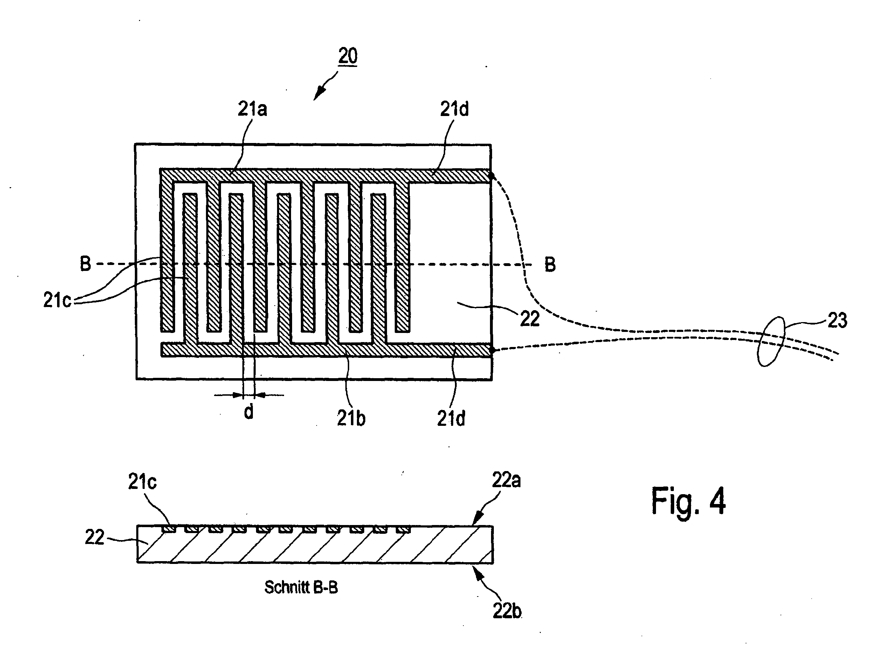

Figur 4 zeigt ein zweites Grundlagenbeispiel der Sensorvorrichtung 20, die als Sensorvorrichtung 1 in allgmeiner Weise in Figur 1 gezeigt ist. Figur 4 zeigt eine Draufsicht auf die Anordnung der Sensorvorrichtung 20, sowie eine Schnittdarstellung entlang einer Schnittlinie B-B.

Die Sensorvorrichtung 20 gemäß dem zweiten Grundlagenbeispiel umfasst erste und zweite Elektroden 21a und 21 b, die auf einer gemeinsamen Grundplatte 22 angeordnet sind. Gemäß der Darstellung in Figur 4 ist in schematischer Weise die aus einem isolierenden Material bestehende Grundplatte 22 als eine rechteckförmige Platte angeordnet.

Die Grundplatte 22 kann auch eine davon abweichende Form aufweisen.

Die erste und zweite Elektrode 21a und 21b sind in der Weise flächig auf der Grundplatte 22 angeordnet, dass diese von einer Seite ausgehend einzelne Elektrodenarme 21 c aufweisen, die auf der Grundplatte 22 abwechselnd angeordnet sind. Die erste und zweite Elektrode 21a und 21b greifen kammförmig ineinander, wobei jedoch sämtliche Teile der beiden Elektroden 21a und 21b voneinander um einen vorbestimmten Abstand d beabstandet sind. Auf der aus isolierendem Material bestehenden Grundplatte 22 sind somit die beiden Elektroden 21a und 21b elektrisch von einander getrennt und jeweils elektrischen einem Pol zugeordnet. Die beiden Elektroden 21a und 21 b bilden einen kapazitiven Sensor und sind auf einer ersten Seite oder ersten Oberfläche 22a der Grundplatte 22 angeordnet. Eine gegenüber liegende Oberfläche wird als zweite Oberfläche 22b bezeichnet.

Die beiden Elektroden 21a und 21b weisen jeweilige Anschlussbereiche 21d auf, mit denen entsprechende Anschlussleitungen 23 verbunden sind. Die Anschtussteitungen 23 können auf der ersten oder der zweiten Oberfläche 22a oder 22b angeordnet sein. In gleicherweise wie beim ersten Grundlagenbeispiel kann ein Kondensator C zwischen die erste Elektrode 21a und die zweite Elektrode 21 b und somit zwischen den elektrischen Polen angeordnet sein.

Figur 4 zeigt des Weiteren eine Schnittansicht der Sensorvorrichtung 20 gemäß dem zweiten Grundlagenbeispiel entlang der Schnittlinie B-B. Hieraus ist erkennbar, dass die einzelnen Teile der beiden Elektroden 21a und 21 b versenkt und damit bündig mit der ersten Oberfläche 22a in der isolierenden Grundplatte 22 angeordnet sind. Es können die Elektrodenteile der beiden Elektroden 21a und 21b auch teilweise in der Grundplatte 22 versenkt sein, oder können auf der Grundplatte 22 und insbesondere auf der ersten Oberfläche 22a derselben angeordnet sein. In jedem Fall sind sämtliche Teile der ersten Elektroden 21a um einen vorbestimmten Abstand d zur zweiten Elektrode 21b beabstandet. Der Bereich zwischen den Elektroden 21a und 21b und somit auch zwischen den Elektrodenarmen 22c verläuft auf der ersten Oberfläche 22a der Grundplatte 22 mäanderformig.

### Drittes Gundlagenbeispiel

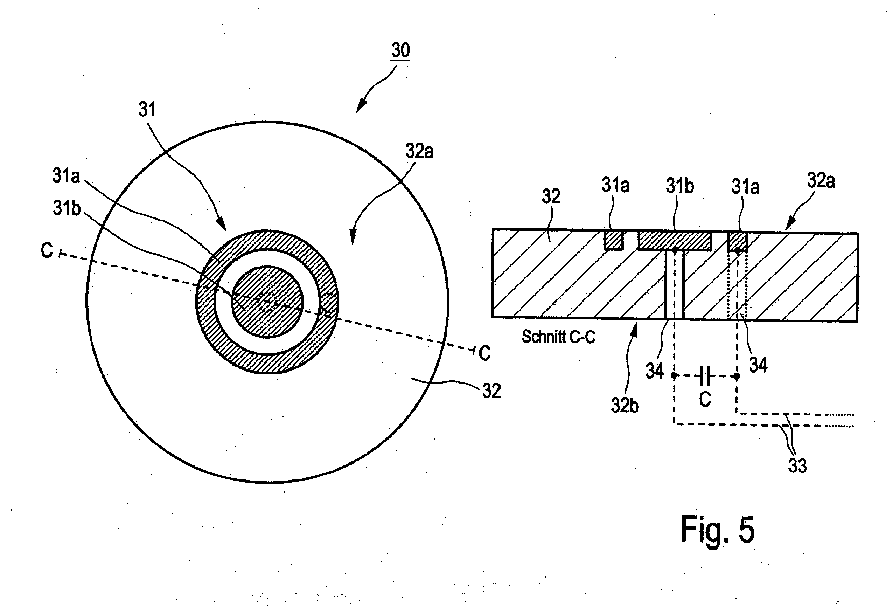

Ein drittes Grundlagenbeispiel der Sensorvorrichtung 30 wird nachstehend in Verbindung mit Figur 5 beschrieben.

Die in Figur 5 dargestellte Sensorvorrichtung 30 ist in gleicher Weise wie die Sensorvorrichtungen (10 und 20) gemäß dem ersten und zweiten Grundtagenbeispiel als kapazitiver Sensor ausgebildet. Die Sensorvorrichtung 30 gemäß dem dritten Grundlagenbeispiel umfasst Elektroden 31, bestehend aus einer äußeren Elektroden 31 a und einer inneren Elektrode 31b. Die innere Elektroden 31b ist bevorzugt als eine Kreisfläche mit einem vorbestimmten Durchmesser aufgebaut, während die äußere Elektrode 31a kreisringförmig um die innere Elektrode 31 b und beabstandet zu dieser angeordnet ist. Beide Elektroden 31 sind auf einer Grundplatte 32 aus einem isolierenden Material und dort auf einer ersten Oberfläche 32a der Grundplatte 32 angeordnet und befestigt und elektrisch voneinander isoliert. Der inneren und äußeren Elektrode 31 a und 31 b ist jeweils elektrisch ein Pol zugeordnet.

Die Grundplatte 32 ist gemäß dem vorliegenden dritten Grundlagenbeispiel nach Figur 5 als im Wesentlichen kreisförmige Scheibe mit einem vorbestimmten Durchmesser ausgebindet, der größer ist als der äußere Durchmesser der kreisringförmigen äußeren Elektrode 31a. Es können die Elektroden 31 bündig mit der ersten Oberfläche 32a der Grundplatte 32 angeordnet und somit in diese eingesetzt sein, oder es können die Elektroden 31 ganz oder teilweise außerhalb des Materials der Grundplatte 32 auf der ersten Oberfläche 32a angeordnet sein. Der erste Fall ist in der neben der Draufsicht angeordneten Schnittdarstellung entsprechend der Schnittlinie C-C angegeben.

Die auf der Grundplatte 32 angeordneten Elektroden 31 sind mit entsprechenden Anschlussleitungen 33 verbunden. Hierzu weist die Grundplatte 32 im Bereich jeder der beiden Elektroden 31 und deren Anschlüsse an die Anschlussleitungen 33 entsprechende Öffnungen 34 in einer der ersten Oberfläche 32a gegenüber liegenden zweiten Oberfläche 32b auf, in denen die Anschlussleitungen 33 angeordnet sind. Die Anschlussleitungen 33 werden zur zweiten Oberfläche 32b der Grundplatte 32 und nach außen geführt. Hierbei werden die Anschlussleitungen weiter zu dem in Figur 1 gezeigten Ansteuerungsteil 5 geführt, mittels dessen entsprechende Ströme und ¹H Spannungen mit vorbestimmten Frequenzen an die Elektroden 31 angelegt werden Ein Kondensator C karin zwischen die Elektroden 31 und damit zwischen die unterschiedlichen elektrischen Pole geschaltet werden.

Die Grundplatte 32 besteht aus einem isolierenden und festen Material, wobei die scheibenförmige Ausbildung lediglich beispielhaft ist. Es kann auch eine beliebige andere flächige Form für die Grundplatte 32 gewählt werden. In jedem Fall ist eine ausreichend ebene Fläche (erste Oberfläche 32a) zur Ausbildung der Elektroden 31 vorzusehen.

In Figur 5 sind im linken Teil in der Draufsicht die beiden Elektroden 31a und 31b als kreisringförmige oder scheibenförmige Anordnung dargestellt. Es kann beispielsweise die innere Elektrode 31 b auch als ein Quadrat, Dreieck oder Rechteck ausgebildet sein, wobei in diesem Fall die äußere als eine Ringstruktur ausgebildete Elektrode 31 a der Kontur der inneren Elektrode 31 b folgt und um einen vorbestimmten Abstand zu dieser beabstandet ist. Mit der isolierenden Grundplatte 32 sind somit die beiden Elektroden 31a und 31b voneinander elektrisch isoliert.

Wird die Sensorvorrichtung 30 gemäß dem dritten Grundlagenbeispiel in einem Behälter wie dem Behälter 2 und vorzugsweise in einer Seitenwand im Bodenbereich oder einer Bodenwand des Behälters 2 angeordnet, dann wird die Grundplatte 32 gegenüber der Behälterwand abgedichtet, sodass kein Medium 3 im Bereich der eingesetzten Grundplatte 32 der Sensorvorrichtung 30 unerwünscht austritt.

### Abwandlungen des ersten bis dritten Grundlagenbeispiels

Hinsichtlich der vorstehend beschriebenen Grundlagenbeispiele 1 bis 3 ist jeweils eine Grundplatte 12, 22 oder 32 vorgesehen, die aus einem festen isolierenden Material besteht. Hierzu eigenen sich verschiedene Kunststoffe, Glas oder Glaskeramik. Das Grundmaterial der Grundplatte 12, 22 und 32 sowie auch der Grundplatten der nachfolgend beschriebenen Ausführungsbeispiele muss eine hohe Wasserbeständigkeit besitzen, wobei möglichst wenig Wasser durch das Material selbst aufgenommen wird. Zur Verminderung des Eindringens von Wasser oder weiteren Komponenten des Mediums 3 im Behälter 2 wird das Grundmaterial, d.h. die Oberflächen der Grundplatte geglättet und poliert, Ferner muss das Grundmaterial der Grundplatte 12, 22 und 32 möglichst stabile Eigenschaften in elektrischer Hinsicht aufweisen, wie beispielsweise die Isolationseigenschaften und eine möglichst stabile Dielektrizitätszahl. Des Weiteren ist es erforderlich, dass das Grundmaterial der Grundplatte 12, 22 und 32 eine vorbestimmte Festigkeit aufweist, sodass die Ausbildung einer Grundplatte in der vorstehend beschriebenen Form gewährleistet, dass diese mechanisch sicher in einer Öffnung des Behälters 2 in der Behälterwand vorzugsweise nahe dem Behälterboden oder im Behälterboden selbst eingebaut werden kann und langfristig und stabil eine Abdichtung gewährleistet ist. Dies ist insbesondere im Hinblick auf den Betrieb einer Wascheinrichtung zu berücksichtigen, bei der in Verbindung mit entsprechend hohen Schleuderdrehzahlen der Waschtrommel starke Vibrationen auftreten können.

Die in den Figuren abgebildeten Elektroden 11, 21 oder 31 können im Fall des ersten Grundlagenbeispiels aus Edelstahl gebildet werden, wobei nach einer entsprechenden Vorbehandlung die beiden äußeren Elektroden 11 a und die innere Elektrode 11 b mit einem elektrochemisch inerten Material, wie beispielsweise Gold, beschichtet werden. Die Beschichtung kann beispielsweise mittels Sputtern erfolgen. Hierbei können die Elektroden ganz oder zumindest teilweise mit dem inerten Material (wie z. B. Gold) beschichtet werden.

Es ist erforderlich, dass alle Oberfläche der Elektroden 11, 21 oder 31, im Falle des ersten Grundlagenbeispiels sämtliche mit dem Medium 3 im Behälter 2 in Berührung kommende Flächen, so glatt wie möglich ausgeführt sind, um jegliches Eindringen auch geringer Mengen von Wasser oder weiterer Substanzen des Mediums 3 in das Material der Elektroden 11, 21 oder 31 zu vermeiden.

Das Grundmaterial der Elektroden 11, 21 oder 31 in sämtlichen Gundlagenbeispielen kann beispielsweise auch in Form einer Keramik oder Glaskeramik bereit gestellt werden, wobei die Oberfläche der aus diesen Werkstoffen gebildeten Teile und Komponenten (Elektroden und/oder Grundplatte) geschliffen und auf Hochglanz poliert wird, und es kann nach einer entsprechenden Behandlung der polierten Oberflächen eine Schicht mit einem inerten und elektrisch leitenden Material, z. B. eine Goldschicht, aufgetragen werden. Dies kann beispielsweise mittels Sputtern erfolgen.

Mit dem entsprechenden Gestalten der Oberflächen beispielsweise der Elektroden 11, 21 und 31 (und auch der Grundplatte 12, 22 und 32) kann das Eindringen von Wasser oder weiteren Inhaltsstoffen des Mediums 3 vermieden werden,
so dass eine ungünstige Beeinflussung von Messergebnissen durch eine Veränderung der elektrischen und auch der mechanischen Eigenschaften der Elektroden 11, 21 oder 31 und auch der Grundplatte 12, 22 oder 32 vermieden werden kann.

Im Falle des ersten Grundlagenbeispiels (Figuren 2 und 3) können die Elektroden 11a und 11 b aus einer Keramik oder Glaskeramik in der entsprechenden halbschalenförmigen Ausgestaltung (äußere Elektroden 11a) oder stabförmig (innere Elektrode 11 b) gebildet werden. Nach jeweiligen vorbereitenden Maßnahmen erfolgt die Beschichtung mit dem inerten leitfähigen Material, z. B. Gold, und bedarfsweise ein weiteres Polieren. Bei einer einteiligen Ausführung der Elektroden 11 des ersten Grundlagenbeispiels kann die zumindest eine äußere Elektrode sowohl auf ihrer äußeren als auch ihrer inneren (d. h. auf den dem Medium ausgesetzten Oberflächen) mit dem inerten Material beschichtet werden. Zumindest ist die innere Oberfläche der zu zumindest einen äußeren Elektrode 11a, die der inneren Elektrode 11b gegenüber liegt, mit dem inerten Material zu beschichten und mittels der Anschlussleitungen galvanisch zu verbinden. Dies gilt auch bei der alternative Ausführung der äußeren Elektrode des ersten Grundlagenbeispiels in Form eines (mit Öffnungen versehenen) vollständigen Höhlzylinders, wobei hier zumindest die Innenseite (gegenüber der inneren Elektrode 11b) beschichtet ist. Die Anschlussleitungen 13 werden im beschichteten Bereich mit den jeweiligen Elektroden 11a und 11b verbunden, wobei bei mehrteiligen äußeren Elektroden 11a sämtliche Teile der äußeren Elektroden 11a mit demselben Anschluss (Pol) verbunden werden.

Im Falle der flächig ausgebildeten Elektroden gemäß den Sensorvorrichtungen 20 und 30 des zweiten und dritten Grundlagenbeispiels besteht die Möglichkeit, nach einer entsprechenden Vorbehandlung der Oberfläche (zumindest der jeweiligen ersten Oberfläche 22a und 32a) der jeweiligen Grundplatte 22 und 32 das Muster und die Ausgestaltung der jeweiligen Elektroden 21 und 31 mittels eines elektrochemisch inerten Materials wie beispielsweise Gold zu bilden. Hierbei kommt ebenfalls die Möglichkeit des Sputterns in Betracht. Auch können die Elektroden 21 und 31 des zweiten und dritten Grundlagenbeispiels aus einem in der Grundplatte 22 und 23 ganz oder teilweise versenkten metallischen Leiter gebildet werden, der mit einem inerten leitenden Material (z. B. Gold) beschichtet sein kann.

In den Figuren sind die jeweiligen Elektroden 11, 21 und 31 mittels entsprechender Anschlussleitungen 13, 23 oder 33 verbunden, wobei diese ihrerseits wieder mit dem in Figur 1 gezeigten Ansteuerungsteil 5 verbunden sind. Da die Elektroden 11, 21 oder 31 gemäß der vorstehenden Beschreibung bevorzugt aus einem elektrochemisch inerten Material bestehen oder zumindest mit diesen beschichtet sind (wie beispielsweise Gold) ist es sinnvoll, keine weiteren unterschiedlichen Metalle innerhalb der jeweiligen Sensorvorrichtung 10, 20 oder 30 zu verwenden. Bevorzugt werden daher die zumindest mit Gold beschichteten Elektroden, mit Golddrähten kontaktiert zur Bildung der erforderlichen Anschlussleitungen 13, 23 und 33. Es kann die Kontaktierung durch Bonden oder Löten erfolgen, wobei auch ein leitfähiger Klebstoff verwendet werden kann.

Grundsätzlich befinden sich die Anschlussleitungen 13, 23 und 33 der jeweiligen Elektroden nicht im Bereich des Mediums 3, werden somit nicht von dem Medium 3 umströmt, da hierdurch eine Beeinflussung des Mediums oder eine Beeinflussung der Anschlussleitungen und der Kontaktpunkte sowie der elektrischen Eigenschaften der gesamten Sensoranordnung auftreten kann. Vielmehr sind die Anschlussleitungen 13, 23 und 33 in entsprechende Vertiefungen oder Öffnungen der jeweiligen Grundplatte 12, 22 oder 32 angeordnet und werden auf der Seite der jeweiligen Grundplatte 12, 22 oder 32 (d. h. ausgehend von der zweiten Oberfläche 12a, 22a, 32a) nach Außen geführt, die der mit dem Medium 3 beaufschlagten Seite (der zweiten Oberfläche 12b, 22b, 32b) gegenüber liegt. Falls erforderlich werden auch hier entsprechende Abdichtungsmaßnahmen der Anschlussleitungen 13, 23 und 33 durch temperaturfeste und auch gegenüber dem Medium 3 beständige Materialien wie beispielsweise Kunststoffe vorgenommen.

Für die jeweils mehrteilige Elektrodenanordnung der vorstehend beschriebenen Sensorvorrichtungen 10, 20 und 30 dient somit die jeweilige Grundplatte 12, 22 und 32 als eine Trägerplatte oder ein gemeinsamer Träger. Die Grundplatte 12, 22 oder 32 ist in der vorstehenden Beschreibung der Grundlagenbeispiele einstückig dargestellt. Sie kann jedoch auch aus mehreren miteinander verbundenen und abgedichteten Teilen oder aus verschiedenen Schichten bestehen, oder kann auch aus mehreren der genannten Materialien bestehen, wobei verschiedene Teile aus unterschiedlichen Materialien bestehen. In gleicher Weise wie bei den Elektroden der Sensorvorrichtung 1 können Materialien der Grundplatte 12, 22 und 32 aus Kunststoffen, Keramik oder Glaskeramik ausgewählt werden. Durch eine entsprechende Behandlung der jeweiligen Oberflächen durch Glätten und Polieren kann das Eindringen von Fremdstoffen in das Material der Grundplatte 12, 22 und 32 vermieden werden. Dies betrifft insbesondere die dem Medium 3 ausgesetzten Oberflächen. Die Materialien können auch miteinander verbunden werden, so dass beispielsweise eine Grundplatte aus mehreren Schichten unterschiedlichen Materials gebildet werden kann.

Bei der Anwendung der verschiedenen Grundlagenbeispiele der Sensorvorrichtung gemäß der vorstehenden Beschreibung besteht des weiteren die Möglichkeit, auch beispielsweise Frischwasser in Wasseraufbereitungsanlagen, oder auch Abwässer von Haushalten oder Industrie auf einen Schadstoffgehalt zu untersuchen oder auch erwünschte oder unerwünschte Konzentrationsänderungen von Substanzen im Wasser zu erfassen. Dies kann für eine Wasserversorgung oder im Hinblick auf Reinigungsvorgänge wässriger Lösungen sinnvoll sein. Mit den erfassten Werten kann eine Steuerung oder Regelung durchgeführt werden. Auch können Kühlmedien auf der Basis wässriger Lösungen im Hinblick auf die Konzentration von Zusatzstoffen in der Lösung überprüft werden.

Die jeweiligen Sensorvorrichtungen sind derart in dem Behälter 2, und beispielsweise innerhalb einer Wascheinrichtung, angeordnet, dass diese vom Medium 3 umströmt werden und in Folge der entsprechenden Strömung unerwünschte Ablagerungen an den Sensorvorrichtungen vermieden werden. In Figur 1 zeigt der Pfeil P die Möglichkeit der Beeinflussung des Waschvorgangs der Wascheinrichtung in dem Behälter 2 mit in Verbindung mit dem Medium 3 durch die Hauptsteuereinheit 6, die sowohl mit einem internen Programm mit vom Benutzer eingestellten Parametern als auch mit Erfassungsergebnissen der Steuerungseinrichtung 4 den Waschvorgang steuert oder egelt.

### Ausführungsbeispiel der Erfindung

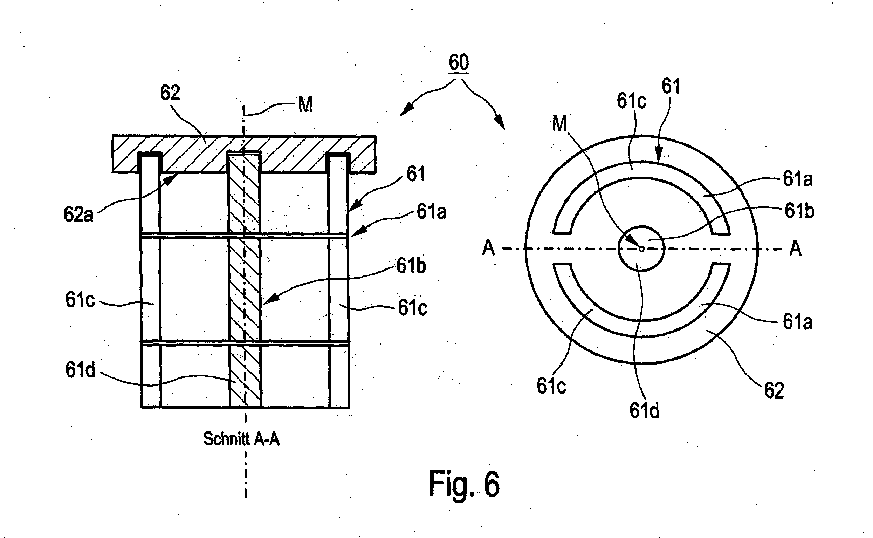

Nachstehend wird unter Bezugnahme auf die Figuren 6, 7 und 8 ein Ausführungsbeispiel der vorliegenden Erfindung beschrieben.

Fig. 6 zeigt die Anordnung einer Sensorvorrichtung 60, wobei im Wesentlichen ein Zylinderkondensator aufgebaut ist, wie er in ähnlicher Weise in Verbindung mit dem ersten Grundlagenbeispiel beschrieben wurde. Die Sensorvorrichtung 60 ist beispielsweise in dem Behälter 2 gemäß Figur 1 angeordnet und dem zu erfassenden Medium 3 zumindest teilweise ausgesetzt.

Auf einer Grundplatte 62 aus einem isolierenden Material sind Elektroden 61 angeordnet, bestehend aus zumindest einer Außenelektrode 61a (oder äußere Elektrode) und einer Innenelektrode 61b (oder innere oder Mittelelektrode). Im vorliegenden Ausführungsbeispiel sind zwei Außenelektroden 61a vorgesehen, die konzentrisch und mit Lücken um die Innenelektrode 61 b angeordnet sind, so dass einerseits ein Zylinderkondensator gebildet wird und andererseits die Elektroden 61 a und 61 b innerhalb und außerhalb der Außenelektrode 61a von dem zu erfassenden Medium 3 zumindest teilweise umströmt werden können. Die Grundplatte 62 kann beispielsweise aus einem Kunststoff, aus Glas, aus Glaskeramik oder einer Keramik bestehen.

Im Unterschied zu der Elektrodenanordnung der Sensorvorrichtung 10 (zur Bildung eines Zylinderkondensators) gemäß dem ersten Grundlagenbeispiel (Figuren 2 und 3) sind, die jeweiligen Elektroden 61 a und 61 b gemäß dem sechsten Ausführungsbeispiel in einer Erstreckungsrichtung entlang einer Linie M mehrteilig ausgeführt. Hierbei weist die zumindest eine Außenelektrode 61a (oder beide um die Innenelektrode (Mittelelektrode) 61 b konzentrisch angeordnete Außenelektroden 61 a) eine entlang der Linie M gleichartige Aufteilung wie die Mittelelektrode 61 b auf. Die aus einem isolierenden Material bestehende und vorzugsweise ebene Grundplatte 62 dient hierbei als isolierende Halteeinrichtung für die Sensorvorrichtung 60 und speziell der Elektroden 61a und 61b.

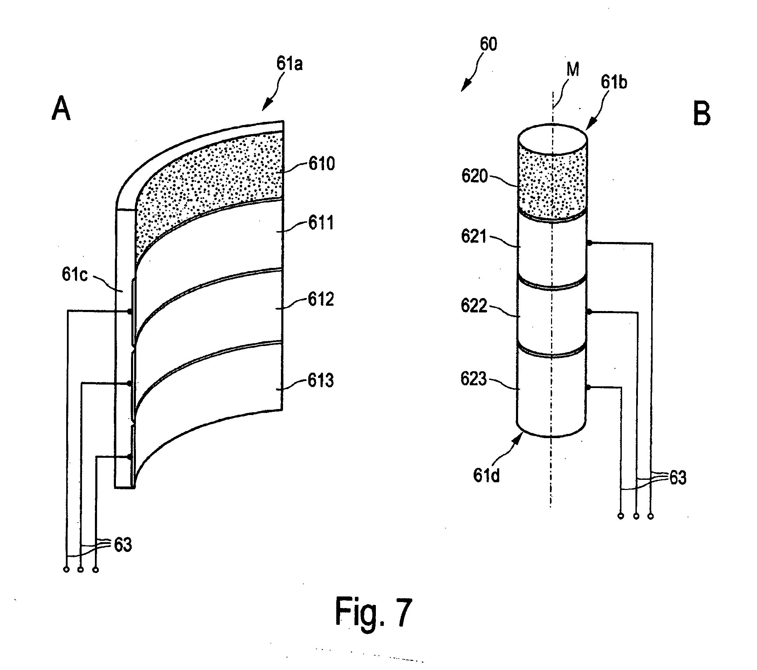

Die vorstehend beschriebene mehrteilige Ausführung der jeweiligen Elektroden 61 a und 61b gemäß dem sechsten Ausführungsbeispiel in der Erstreckungsrichtung entlang der Linie M kann dabei auf eine erste Weise erfolgen, indem die zumindest eine Außenelektrode 61a auf einem Zylindersegment 61c (mit einer in der zweidimensionalen Abwicklung quadratischen und bevorzugt rechteckigen Fläche) ausgebildet ist, das aus einem Stück eines nichtleitenden Materials besteht. Als ein Material für das Zylindersegment 61 c kann die Auswahl für die jeweilige Grundplatte der vorherigen Grundlagenbeispiele verwendet werden. Auf dem einstückigen Zylindersegment 61 c können einzelne Teile der äußeren Elektrode 61a flächig und gegeneinander isoliert ausgebildet werden. Die einstückige Ausführung-des Zylindersegments 61 c der zumindest einen äußeren Elektrode ist in der nachstehend noch beschriebenen Figur 7A beispielhaft dargestellt.

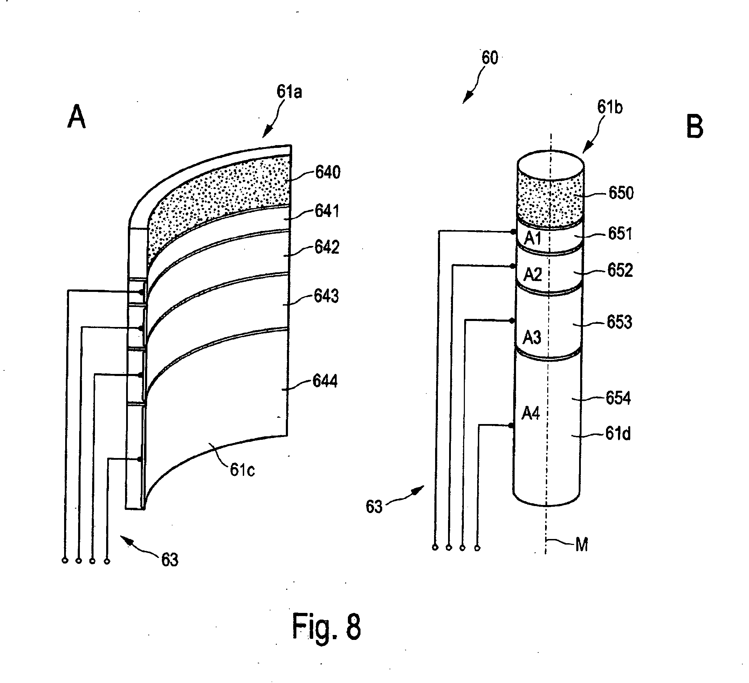

Gemäß der Darstellung in den Figuren 6 und 8 kann die mehrteilige Ausführung der jeweiligen Elektroden 61a und 61b gemäß dem Ausführungsbeispiel in der Erstreckungsrichtung entlang der Linie M auf eine zweite Weise erfolgen, indem beispielsweise die zumindest eine Außenelektrode 61 a auf einem mehrteilig ausgeführten Zylindersegment 61 c aufgebaut ist. Die einzelnen Teile des mehrteiligen Zylindersegments 61 c entsprechen dabei den jeweiligen Teilen bzw. aktiven Flächen der Elektrode 61a in der Anordnung entlang der Linie M. Die jeweiligen aktiven Flächen werden nachstehend als Elektrodenabschnitte bezeichnet.

Die vorstehende beschriebenen einstückige oder mehrteilige Ausführung der in Verbindung mit einem Zylindersegment 61c ausgebildeten zumindest einen Außenelektrode 61a trifft in gleichartiger Weise auch auf die Innenelektrode (Mittelelektrode) 61 b zu. Gemäß Figur 6 kann die Innenelektrode 61 b aus einem aus einem isolierenden Material (entsprechend der Grundplatte 62) bestehenden einstückigen Stab oder Hohlzylinder 61 d gebildet werden. Alternativ kann der Stab oder Hohlzylinder 61 d auch mehrteilig ausgeführt sein, wie es in den Figuren 6 bis 8 dargestellt ist. Die jeweiligen aktiven Flächen der Innenelektrode 61 b (nachstehend als Elektrodenabschnitte bezeichnet) sind auf dem einstückigen oder mehrteiligen Stab oder Hohlzylinder 61 d aufgebracht und gegeneinander isoliert.

Weitere Einzelheiten zur Aufteilung der Elektroden 61 der Sensorvorrichtung 60 sind in den Figuren 7 und 8 gezeigt.

Fig. 7A zeigt Einzelheiten zur Außenelektrode 61 a und Fig. 7B zeigt Einzelheiten zur Innenelektrode 61b. Wie es in Verbindung mit dem ersten Grundlagenbeispiel gemäß Fig. 2 gezeigt ist, sind zum Ansteuern der jeweiligen Elektroden 61 Anschlussleitungen 63 vorgesehen. Diese sind in den Figuren 7 und 8 angedeutet und in Fig. 6 zur Vereinfachung der Darstellung weggelassen.

Fig. 7A zeigt die zumindest eine Außenelektrode 61a, wobei in dieser Figur von oben beginnend ein erster Teil der Außenelektrode 61 a als Befestigungsabschnitt 610 vorgesehen ist und zur Befestigung der Außenelektrode 61 a in der Grundplatte 62 dient.

In Richtung der Erstreckung der äußeren Elektrode 61a entlang der Linie M sind beispielsweise ein erster Elektrodenabschnitt 611, ein zweiter Elektrodenabschnitt 612 und ein dritter Elektrodenabschnitt 613 vorgesehen. Die Elektrodenabschnitte 611 bis 613 stellen jeweils Teile der äußeren Elektrode 61a dar und sind einzeln (getrennt, individuell) mittels der entsprechenden Anschlussleitungen 63 ansteuerbar. Die jeweiligen Elektrodenabschnitte 611 bis 613 sind auf einem gemeinsamen Elektrodenkörper in Form des Zylindersegments 61 c der äußeren Elektrode 61a ausgebildet und gegeneinander isoliert.

In ähnlicher Weise ist die innere Elektrode 61 b gemäß Fig. 7B aufgeteilt. Die innere , Elektrode 61b umfasst von oben nach unten gesehen gemäß Fig. 7B einen Befestigungsabschnitt 620 sowie aufeinanderfolgende Elektrodenabschnitte'621, 622 und 623, die jeweils mit Anschlussleitungen 63 verbunden sind.

Die äußere Elektrode 61 a und die innere Elektrode 61 b sind somit in etwa gleichartiger Weise aufgeteilt, wobei die jeweiligen Elektrodenabschnitte 611 bis 613 und 621 bis 623 in der Erstreckungsrichtung gemäß der Linie M gleiche Größe aufweisen. Die Elektrodenabschnitte 621 bis 623 sind ebenfalls gegeneinander isoliert.

Durch die Anordnung der gemäß den Figuren 7A und 7B aufgebauten äußeren und inneren Elektrode 61a und 61b der Sensorvorrichtung 60 werden innerhalb der im Wesentlichen als Zylinderkondensator ausgebildeten Sensorvorrichtung 60 jeweilige Einzelkapazitäten gebildet, wobei einander entsprechende Elektrodenabschnitte 611 bis 613 der äußeren Elektrode 61a den Elektrodenabschnitten 621 bis 623 der inneren Elektrode 61b gegenüberliegen. Eine Ansteuerung mittels der entsprechenden Zuleitungen 63 ermöglicht das Verwenden sämtlicher Elektrodenabschnitte oder einzelner Elektrodenabschnitte 611 bis 613 und 621 bis 623 zur Durchführung einer Messung. Es ist hierbei eine Anpassung der Kapazitäten und damit der Impedanzen an vorbestimmte Bedingungen möglich.

Fig. 8 zeigt eine alternative Ausführungsform des Ausführungsbeispiels in Verbindung mit der Sensorvorrichtung 60. In gleichartiger Weise sind die äußere Elektrode 61a und die innere Elektrode 61b in verschiedene Abschnitte aufgeteilt. Ein erster Abschnitt der äußeren Elektrode 61a dient als Befestigungsabschnitt 640 zur Befestigung in der Grundplatte 62 (Fig. 6). Die äußere Elektrode 61 b umfasst ferner erste bis vierte Elektrodenabschnitte 641, 642, 643 und 644, die sich entlang der Linie M erstrecken. Die Elektrodenabschnitte 641 bis 644 sind in gleicher Weise auf einen gemeinsamen (einstückigen oder mehrteiligen) Träger der äußeren Elektrode 61a in Form des Zylindersegments 61c aufgebaut und jeweils gegeneinander isoliert, so dass sie individuell.(getrennt) oder gemeinsam über Anschlussleitungen 63 angesteuert werden können.

Die innere Elektrode 61 b gemäß Fig. 8B weist einen zur äußeren Elektrode 61a korrespondierenden Aufbau auf, wobei ein Befestigungsabschnitt 650 zur Befestigung in der Grundplatte 62 dient. Weitere Elektrodenabschnitte 651, 652, 653 und 654 erstrecken sich auf der inneren Elektrode 61b entlang der Linie M. Die jeweiligen Elektrodenabschnitte 651 bis 654 sind gegeneinander isoliert angeordnet und können mittels der damit verbundenen jeweiligen Anschlussleitungen 63 individuell oder gemeinsam von außen angesteuert werden.

Im Vergleich zu der Anordnung der jeweiligen Elektrodenabschnitte 611 bis 613 und 621 bis 623 gemäß den Figuren 7A und 7B sind die Elektrodenabschnitte 641 bis 644 und 651 bis 654 gemäß der Darstellung in den Figuren 8A und 8B von unterschiedlicher Ausdehnung in der Erstreckungsrichtung entlang der Linie M: In der Darstellung der Figuren 8A und 8B weist beispielsweise der jeweilige erste Elektrodenabschnitt 641 und 651 die geringste Ausdehnung auf, während der vierte Elektrodenabschnitt 644 und 654 die größte Ausdehnung in Richtung der Linie M aufweist. Die Längsausdehnung der jeweiligen Elektrodenabschnitte sind bei der äußeren Elektrode 61a und der inneren Elektrode 61b jeweils korrespondierend, so dass mittels einer entsprechenden Ansteuerung jeweiliger entsprechenden Elektrodenabschnitte über die individuellen Anschlussleitungen 63 jeweils Teilkapazitäten (Impedanzen) eines Zylinderkondensators betrachtet werden können, die in Abhängigkeit von der Größe der jeweils korrespondierenden Elektrodenabschnitte 641 bis 644 und 651 bis 654 unterschiedliche Kapazitätswerte und damit auch unterschiedliche Impedanzen aufweisen.

Im Einzelnen stellt bei der äußeren Elektrode 61a gemäß den Figuren 7A und 8A der einzelne Elektrodenabschnitt 611 bis 613 und 641 bis 644 einen Teil einer inneren Zylinderwand dar, dem eine bestimmte Fläche (aktive Fläche) zugeordnet werden kann. In ähnlicher Form kann den ringförmig ausgebildeten Elektrodenabschnitten 621 bis 623 und 651 bis 654 der jeweiligen inneren Elektrode 61 b der Figuren 7B und 8B eine Ringfläche (aktive Fläche) zugeordnet werden. In Fig. 8B sind für die jeweiligen Ringflächen der Elektrodenabschnitte 651 bis 654 die Flächen A1, A2, A3 und A4 angegeben. Ungeachtet der vereinfachten und schematischen Darstellung gemäß Fig. 8B stehen die jeweiligen Ringflächen A1 bis A4 zueinander in einem vorbestimmten Zusammenhang. Beispielsweise kann die Ringfläche A4 des vierten Elektrodenabschnitts 654 das Doppelte der Ringfläche A3 des dritten Elektrodenabschnitts 653 sein. Ferner kann die Ringfläche A3 des dritten Elektrodenabschnitts 653 das Doppelte der Ringfläche A2 des zweiten Elektrodenabschnitts 652 sein. In gleicher Weise kann die Ringfläche A2 des zweiten Elektrodenabschnitts 652 beispielsweise das Doppelte der Ringfläche A1, des ersten Elektrodenabschnitts 651 sein.

Gemäß der Beziehung A4=2xA3=4xA2=8xA1 können sich somit mögliche relative Verhältnisse zwischen den ringförmigen Flächen A1 bis A4 am Beispiel der inneren Elektrode 61 b ergeben. Dies trifft in gleichartiger Weise auch auf die Flächenverhältnisse der äußeren Elektrode 61 a (Teilflächen der Elektrodenabschnitte 641 und 644) zu. Die Erfindung ist nicht auf die beispielhafte Darstellung ganzzahlige Verhältnisse festgelegt, viel mehr können auch andere Verhältnisse der Flächen der Elektrodenabschnitte (Verhältnisse der aktiven Flächen) bestimmt werden.

Es ist auf diese Weise möglich, mittels der Sensorvorrichtung 60 in Form eines Zylinderkondensators Teilkapazitäten in Abhängigkeit von der jeweiligen Größe der Ringflächen A1 bis A4 oder der aktiven Flächen zu bilden, wobei die Teilkapazitäten und damit auch die Impedanzen zueinander in einem vorbestimmten Verhältnis stehen können.

In den Figuren 7A, 7B, 8A und 8B sind die jeweiligen Anschlussleitungen 63 schematisch eingezeichnet und mit den jeweiligen Elektrodenabschnitten 611 bis 613, 621 bis 623, 641 bis 644 und 651 bis 654 verbunden. Die Anschlussleitungen 63 können im Außenbereich der jeweiligen inneren oder äußeren Elektrode 61 a und 61 b geführt werden, oder können im Inneren des Trägermaterial (Zylindersegment 61c und Stab oder Hohlzylinder 61d) der jeweiligen Elektrode geführt werden, so dass die äußere und damit wirksame Oberfläche der jeweiligen Elektrodenabschnitte nicht durch den Verlauf von Anschlussleitungen 63 beeinträchtigt ist.

Mit den verschiedenen Elektrodenabschnitten 611 bis 613, 621 bis 623, 641 bis 644 und 651 bis 654 werden jeweilige Teilsensoren (Teilkapazitäten, Teilimpedanzen). gebildet, die gemäß den vorstehenden Angaben getrennt angesteuert werden können, so dass sie einzeln, in Gruppen oder insgesamt zur Durchführung einer Messung herangezogen werden können. Eine Anpassung an die zu erwartenden Eigenschaften eines zu messenden Mediums 3 oder an eine Messvorrichtung oder Auswertungseinrichtung ist auf diese Weise möglich. Es können daher in Verbindung mit einem zu erfassenden Medium 3 und einer Auswertung mit den vorstehend beschriebenen Anordnungen unterschiedliche Empfindlichkeiten der jeweiligen Sensoreinrichtung eingestellt werden.

Die jeweiligen vorstehend angegebenen Elektrodenabschnitte 611 bis 613, 621 bis 623, 641 bis 644 und 651 bis 654 der Sensorvorrichtung 60 gemäß dem Ausführungsbeispiel in beiden Varianten können aus einem gleichen Material, wie beispielsweise Gold, Platin, Chrom oder Rhodium (und dergleichen) gebildet werden, wobei die Beschichtung mittels Sputtern oder einem anderen geeigneten Verfahren erfolgen kann. Es können auch jeweilige Elektrodenabschnitte gegenüber anderen Elektrodenabschnitten aus einem unterschiedlichen Material bestehen, wobei eine beliebige Kombination von Materialien möglich ist. Beispielsweise kann der erste Elektrodenabschnitt 651 aus Gold, der zweite Elektrodenabschnitt 652 aus Platin, der dritte Elektrodenabschnitt 653 aus Rhodium und der vierte Elektrodenabschnitt 654 aus Chrom bestehen, wobei die jeweiligen Metalle auf das Trägermaterial der inneren Elektrode 61 (Fig. 8B) aufgebracht sind.

Auf diese Weise ist es möglich, die kapazitiven Eigenschaften und weitere Eigenschaften der jeweiligen Teilsensoren (Teilkapazitäten) sowie damit der gesamten Sensorvorrichtung 60 entsprechend den Materialien zu beeinflussen oder zu ändern, wobei bestimmte Materialien für bestimmte Ionen angepasst werden können. Auch können die verschiedenen Elektrodenabschnitte eine gleiche oder unterschiedliche Oberflächenbeschaffenheit (wie beispielsweise eine unterschiedliche Rauheit) aufweisen. Insgesamt können unterschiedliche Empfindlichkeiten der Sensorvorrichtung 60 eingestellt werden.

In jedem Fall sind die vorstehend angegebenen aktiven Flächen der Elektrodenabschnitte 611 bis 613, 621 bis 623, 641 bis 644 und 651 bis 654 der Sensorvorrichtung 60 dem zu erfassenden Medium 3 ganz oder zumindest teilweise ausgesetzt. Bevorzugt wird die Anordnung des Zylinderkondensators gemäß Figur 6 vom zu erfassenden Medium 3 durchströmt. Die Oberflächen der jeweiligen aktiven Flächen einzelner Elektrodenabschnitte können des Weiteren spezielle Schutzschichten aufweisen.

Entsprechend der Darstellung im Zusammenhang mit dem vierten Ausführungsbeispiel können somit Teilsensoren gemäß einem Mehr-Bit-System gebildet werden, wobei die Kapazität oder die Impedanz in jeweils einer vorbestimmten Anzahl von Stufen eingestellt und an vorbestimmte Bedingungen angepasst werden kann.

Die Vorrichtung des sechsten Ausführungsbeispiels ist gemäß Fig. 7A und 7B in Verbindung mit jeweils drei Elektrodenabschnitten der äußeren und inneren Elektrode 61 a und 61 b beschrieben. Gemäß Fig. 8A und 8B sind jeweils vier Elektrodenabschnitte gestellt: Die vorliegende Erfindung ist jedoch nicht auf die Anzahl der Elektrodenabschnitte sowie auf die vorstehend angegebenen Flächenverhälthisse festgelegt. Vielmehr können mehr oder weniger Elektrodenabschnitte in jeweils gleichartiger Weise bei der äußeren und inneren Elektrode 61a und 61 b vorgesehen sein.

In der Beschreibung der vorstehenden Grundlagenbeispiele und des Ausführungsbeispiels sind die jeweiligen Elektroden der einzelnen Sensorelemente mit Anschlußleitungen verbunden. Die jeweiligen Anschlußleitungen sind Verbindungen aus elektrisch isolierten Leitungen, wobei die Isolation den Verlauf der Anschlußleitungen außerhalb der Grundplatte als auch den Verlauf auf der Grundplatte betrifft. Außerhalb der Grundplatte sind isolierte Leitungen vorgesehen, während auf der Grundplatte die Anschlußleitungen zu den Elektroden hingeführt und mit diesen verbunden sind. Die elektrische Isolation erfolgt in der Weise, dass die Anschlußleitungen in ihrem gesamten Verlauf auf der Grundplatte mit einer isolierenden Schicht bedeckt sind und lediglich der Bereich der Elektroden hiervon frei ist.

Die vorliegende Erfindung wurde vorstehend in Verbindung mit Ausführungsbeispielen der Sensorvorrichtung beschrieben. Für den auf diesem Gebiet tätigen Fachmann ist es jedoch selbstverständlich, dass die Ausgestaltung der vorliegenden Erfindung gemäß den beschriebenen Figuren und den für die jeweiligen Bauteile und Komponenten verwendeten Bezugszeichen in den Figuren und der Beschreibung sowie die beispielhaften Angaben nicht einschränkend auszulegen sind. Die Erfindung ist auf die angegebene Darstellung in den Figuren und insbesondere auf Dimensionen und Anordnungen sowie Formeln der Komponenten nicht beschränkt. Als zur Erfindung gehörig werden sämtliche Ausführungsformen und Varianten abgesehen, die unter die beigefügten Patentansprüche fallen.

## Patentansprüche

1. Sensorvorrichtung zur Erfassung von Eigenschaften fluider Medien in einem Behälter, wobei die Sensorvorrichtung (60) dem zu erfassenden Medium (3) zumindest teilweise ausgesetzt ist, mit
- einer aus einem isolierenden Material gebildeten Grundplatte (62) mit zumindest einer ersten, den Medium (3) ausgesetzten Oberfläche (62a), und
- zumindest einem als ein Zylinderkondensator ausgebildeten Sensorelement mit zumindest einer äußeren Elektrode (61 a) und einer teilweise von der äußeren Elektrode (61 a) umgebenen inneren Elektrode (61 b), wobei sich die zumindest eine äußere und die innere Elektrode (61a, 61b) von der ersten Oberfläche (62a) der Grundplatte (62) erstrecken und das Medium (3) zwischen die äußere und die innere Elektrode (61a, 61 b) gelangt,
**dadurch gekennzeichnet, dass**
die zumindest eine äußere Elektrode (61a) und die innere Elektrode (61b) in einer Erstreckungsrichtung (M) in eine Mehrzahl gegeneinander isolierter Elektrodenabschnitte (611 bis 613, 621 bis 623, 641 bis 644, 651 bis 654) aufgeteilt ist und die äußere und die innere Elektrode (61a, 61b) in gleicher Weise aufgeteilt sind, und die Elektrodenabschnitte (611 bis 613, 621 bis 623, 641 bis 544, 651 bis 654) der äußeren und inneren Elektroden (61a, 61b) jeweils mit Anschlussleitungen (63) verbunden und individuell und unabhängig voneinander ansteuerbar sind.

2. Sensorvorrichtung nach Anspruch 1, wobei die zumindest eine äußere und die innere Elektrode (61a, 61b) in eine Vielzahl gleicher oder unterschiedlicher Elektrodenabschnitte (611 bis 613, 621 bis 623, 641 bis 544, 651 bis 654) aufgeteilt ist und die bei der ungleichen Aufteilung gebildeten Elektrodenabschnitte (641 bis 644, 651 bis 654) hinsichtlich ihrer Ausdehnung in der Erstreckungsrichtung (M) zueinander in vorbestimmten Verhältnissen aufgeteilt sind.

3. Sensorvorrichtung nach Anspruch 1 oder 2, wobei die einzelnen Elektrodenabschnitte (611 bis 613, 621 bis 623, 641 bis 544, 651 bis 654) aus einem gleichen oder unterschiedlichen leitenden Material gebildet sind, und wobei jeweils einander entsprechende Elektrodenabschnitte der äußeren und der inneren Elektrode (61a, 61b) aus einem gleichen Material gebildet sind.

## Claims

1. A sensor device for detecting properties of fluid media in a container, wherein the sensor device (60) is at least partially exposed to the medium (3) to be detected, comprising
- base plate (62) made of an insulating material and having at least one first surface (62a) exposed to the medium (3), and
- at least one sensor element embodied as a cylindrical capacitor and comprising at least one outer electrode (61 a) and one inner electrode (61 b) partially surrounded by said outer electrode (61 a), wherein the at least one outer and the inner electrode (61 a, 61 b) extend from the first surface (62a) of the base plate (62), and the medium (3) comes between the outer and the inner electrode (61 a, 61b).
**characterized in that**
the at least one outer electrode (61 a) and the inner electrode (61 b) are subdivided in the direction of extension (M) into a plurality of electrode sections (611 to 613, 621 to 623, 641 to 644, 651 to 654) insulated from one another and the outer and the inner electrode (61 a, 61 b) are subdivided in the same manner, and the electrode sections (611 to 613, 621 to 623, 641 to 644, 651 to 654) of the outer and inner electrodes (61 a, 61b) are each connected to connection lines (63) and can be driven individually and independently of each other.

2. The sensor device of claim 1, wherein the at least one outer and the inner electrode (61 a, 61 b) are subdivided into a plurality of identical or different electrode sections (611 to 613, 621 to 623, 641 to 644, 651 to 654) and the electrode sections (641 to 644, 651 to 654) formed by non-identical subdivision are subdivided in respect of their extension in the direction of extension (M) in predetermined ratios in relation to each other.

3. The sensor device of one of the claims 1 or 2, wherein the separate electrode sections (611 to 613, 621 to 623, 641 to 644, 651 to 654) are made of the same conductive material or of different conductive materials, and wherein respectively matching electrode sections of the outer and inner electrodes (61 a, 61 b) are made of the same material.

## Revendications

1. Dispositif de capteur servant à détecter des propriétés de milieux fluides dans un récipient, sachant que le dispositif de capteur (60) est exposé au moins en partie au milieu (3) à détecter, comprenant
- une plaque de base (62) formée à partir d'un matériau isolant, comprenant au moins une première surface (62a) exposée au milieu (3), et
- au moins un élément de capteur réalisé sous la forme d'un condensateur cylindrique comprenant au moins une électrode extérieure (61a) et une électrode intérieure (61b) entourée en partie par l'électrode extérieure (61a), sachant que l'électrode extérieure au moins au nombre de une et l'électrode intérieure (61a, 61b) s'étendent depuis la première surface (62a) de la plaque de base (62) et que le milieu (3) parvient entre l'électrode extérieure et l'électrode intérieure (61a, 61b),
**caractérisé en ce**
**que** l'électrode extérieure (61a) au moins au nombre de une et l'électrode intérieure (61b) sont divisées, dans une direction d'extension (M), en une multitude de segments d'électrode (611 à 613, 621 à 623, 641 à 644, 651 à 654) isolés les uns des autres, et en ce que l'électrode extérieure et l'électrode intérieure (61a, 61b) sont divisées d'une manière identique, et en ce que les segments d'électrode (611 à 613, 621 à 623, 641 à 644, 651 à 654) des électrodes extérieures et intérieures (61a, 61b) sont reliés respectivement à des lignes de raccordement (63) et peuvent être commandés individuellement et séparément les uns des autres.

2. Dispositif de capteur selon la revendication 1, sachant que l'électrode extérieure au moins au nombre de une et l'électrode intérieure (61a, 61b) sont divisées en une pluralité de segments d'électrode (611 à 613, 621 à 623, 641 à 644, 651 à 654) identiques ou différents, et en ce que les segments d'électrode (641 à 644, 651 à 654) formés dans le cadre de la division inégale sont divisés dans des conditions prédéfinies les uns par rapport aux autres au regard de leur allongement dans la direction d'extension (M).

3. Dispositif de capteur selon la revendication 1 ou 2, sachant que les divers segments d'électrode (611 à 613, 621 à 623, 641 à 644, 651 à 654) sont formés à partir d'un matériau conducteur identique ou différent, et sachant que des segments d'électrode, correspondant les uns aux autres respectivement, de l'électrode extérieure et de l'électrode intérieure (61a, 61b) sont formés à partir d'un matériau identique.
